(19) Europäisches Patentamt
European Patent Office
Office européen des brevets

(11) **EP 2 634 560 B1**

## (12) EUROPÄISCHE PATENTSCHRIFT

(45) Veröffentlichungstag und Bekanntmachung des Hinweises auf die Patenterteilung:
**09.09.2020 Patentblatt 2020/37**

(21) Anmeldenummer: **13151867.2**

(22) Anmeldetag: **18.01.2013**

(51) Int Cl.:
**G01N 21/27** *(2006.01)*  **G01N 21/47** *(2006.01)*
**G01N 21/64** *(2006.01)*  **G01N 33/58** *(2006.01)*

(54) **Verfahren zur Bestimmung der Helligkeit eines lumineszenten Teilchens**

Method for determining the brightness of a luminescent particle

Procédé de détermination de la luminosité d'une particule luminescente

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.03.2012 DE 102012101744**

(43) Veröffentlichungstag der Anmeldung:
**04.09.2013 Patentblatt 2013/36**

(73) Patentinhaber: **BAM Bundesanstalt für Materialforschung und -prüfung**
**12205 Berlin (DE)**

(72) Erfinder:
• **Würth, Christian**
**10245 Berlin (DE)**
• **Resch-Genger, Ute**
**12203 Berlin (DE)**
• **Behnke, Thomas**
**13349 Berlin (DE)**
• **Hoffmann, Katrin**
**12524 Berlin (DE)**

(74) Vertreter: **Zimmermann & Partner**
**Patentanwälte mbB**
**Postfach 330 920**
**80069 München (DE)**

(56) Entgegenhaltungen:
**EP-A1- 2 357 465        WO-A1-2009/050536**

DE-A1-102009 006 364

• **CHRISTIAN WÜRTH ET AL: "Polymer-and Glass-based Fluorescence Standards for the Near Infrared (NIR) Spectral Region", JOURNAL OF FLUORESCENCE, KLUWER ACADEMIC PUBLISHERS-PLENUM PUBLISHERS, NE, Bd. 21, Nr. 3, 15. April 2010 (2010-04-15) , Seiten 953-961, XP019919328, ISSN: 1573-4994, DOI: 10.1007/S10895-010-0650-0**
• **ALEXANDRA HUBER ET AL: "Spectroscopic Characterization of Coumarin-Stained Beads: Quantification of the Number of Fluorophores Per Particle with Solid-State 19 F-NMR and Measurement of Absolute Fluorescence Quantum Yields", ANALYTICAL CHEMISTRY, Bd. 84, Nr. 8, 17. April 2012 (2012-04-17) , Seiten 3654-3661, XP055076186, ISSN: 0003-2700, DOI: 10.1021/ac3000682**
• **LAURENT PORRÈS ET AL: "Absolute Measurements of Photoluminescence Quantum Yields of Solutions Using an Integrating Sphere", JOURNAL OF FLUORESCENCE, KLUWER ACADEMIC PUBLISHERS-PLENUM PUBLISHERS, NE, Bd. 16, Nr. 2, 14. Februar 2006 (2006-02-14), Seiten 267-273, XP019400522, ISSN: 1573-4994, DOI: 10.1007/S10895-005-0054-8**
• **CHRISTIAN WÜRTH ET AL: "Comparison of Methods and Achievable Uncertainties for the Relative and Absolute Measurement of Photoluminescence Quantum Yields", ANALYTICAL CHEMISTRY, Bd. 83, Nr. 9, 7. April 2011 (2011-04-07), Seiten 3431-3439, XP055076207, ISSN: 0003-2700, DOI: 10.1021/ac2000303**

**EP 2 634 560 B1**

## Beschreibung

Die Erfindung betrifft ein Verfahren zur Bestimmung der absoluten Helligkeit lumineszenter Teilchen und derenVerwendung für optische Messungen, insbesondere auf dem Gebiet der Kalibrierung der dafür geeigneten Messsysteme.

Im Folgenden werden suspendierte, dispergierte oder aggregierte Teilchen, Kombinationen derartiger Teilchen, Teilchen-Aggregate, Teilchenensembles, Farbstoffmarkierte Teilchen, farbstoffmarkierte Antikörper-Teilchen-Konjugate, enzymkonjugierte Teilchen oder enzymmarkierte Antikörper-Teilchenkonjugate, und jegliche andere Kombinationen lumineszenter Teilchen mit daran gebundenen, adsorbierten, eingeschlossenen oder fest assoziierten weiteren Farbstoffen, Markern, Labeln, Enzymen und/oder Antikörpern als Systeme und die Teilchen aller derartigen Systeme unabhängig von ihrer jeweiligen Form vereinfachend als Teilchen bezeichnet, ohne auf bestimmte Teilchenformen beschränkt zu sein.

Das vorgeschlagene Verfahren betrifft auch die Standardisierung und Referenzierung von optischen Methoden, die lumineszente Teilchen als Analyseplattformen verwenden, sowie die (relative) Quantifizierung aus Fluoreszenzmessungen. Insbesondere betrifft das hier vorgeschlagene Verfahren den Aufbau einer Intensitätsskala, die auf echte physikalische Messgrößen rückführbar ist und die für die Entwicklung und Verwendung von Teilchenstandards auf unterschiedlichsten Einsatzgebieten von Bedeutung ist. Einsatzgebiete derartiger Teilchenstandards sind jegliche Teilchenbasierte lumineszenzbasierte Assays, wie sie z.B. in der Bio- und Umweltanalytik und der medizinischen Diagnostik, der Wirkstoffforschung, der Therapie-Kontrolle oder der Rückstands- und Spurenanalytik etabliert sind. Dementsprechend ist das Verfahren für Hersteller und Anwender von optischen Kontrastmitteln, für Hersteller von lumineszenten, insbesondere von fluoreszenten Teilchen, von Phosphoren oder von Nanoemulsionen sowie für Hersteller und Anwender von Teilchenstandards und Assay-Kits von Interesse.

Unter Teilchenstandards werden nachfolgend verstanden: Standards für Teilchenbasierte Fluoreszenzmessverfahren in Lösung oder im Durchfluss; Standards, die aus sogenannten Teilchen bzw. Beads aufgebaut sind bzw. diese enthalten wie z.B. bead-basierte Array-Standards, z.B. für die Mikroarray-Technologie oder Standards für andere bildgebende Verfahren angewendet werden, z.B. zur Charakterisierung von Gewebe, Gewebeschnitten, Ausstrichen, oder Gelschichten.

Bisher wurden lumineszente oder zur Lumineszenz fähige und im sichtbaren Spektralbereich streuende Teilchen hinsichtlich ihrer optischen Eigenschaften als Pulver oder Suspension charakterisiert, wobei jedoch keine Helligkeitswerte, sondern nur z.B. Fluoreszenzquanten-Ausbeuten bestimmt wurden.

Relative Helligkeitswerte suspendierter Teilchen wurden an einzelnen Teilchen z.B. mittels Durchflusszytometrie oder mittels anderer fluoreszenzmikroskopischer Verfahren ermittelt. Dabei werden keine geräteunabhängigen Werte erhalten, sondern es erfolgten typischerweise nur direkte Vergleichsmessungen zwischen verschiedenen Teilchen-Systemen.

Würth C., Grabolle M., Pauli J., Spieles M. und Resch-Genger U. beschreiben in Analytical Chemistry (2011) 83: 3431-3439 einen Vergleich von Methoden und der jeweils erreichbaren Unbestimmtheit der relativen und der absoluten Messung von Photolumineszenzquantenausbeuten. Würth C., Hoffmann K., Behnke T., Ohnesorge M. und Resch-Genger U. beschreiben in Journal of Fluorescence (2011) 21:953-961 Polymer- und Glas-basierte Fluoreszenzstandards für den Nah-Infrarot (NIR) Spektralbereich. DE 10 2009 006 364 A1 offenbart ein Instrument und ein Verfahren zur quantitativen Messung der Molekularität einer Probe auf der Basis einer Lichtmenge mit Korrelation zur Molekularität.

Die Messung an Einzelteilchen und die dabei gewonnenen Charakteristika sind allein schon aus Gründen der inhomogenen Teilcheneigenschaften eines Ensembles nicht für den Aufbau einer echten quasi-absoluten Intensitätsskala geeignet, die - wie erläutert - beispielsweise für die Entwicklung von Teilchenstandards und für einen Vergleichbarkeit verschiedener Teilchen benötigt wird.

Darüber hinaus wird in der Durchflusszytometrie zur Kalibrierung der Intensitätsachse das sogenannte MESF-System (MESF: measurable fluorescence of equivalent soluble fluorophore) eingesetzt. Dementsprechend kommen Teilchen mit jeweils den Teilchen zum Zweck der relativen Quantifizierung zugeordneten MESF-Werten zum Einsatz. Kalibrierbeads mit "zugeordneten MESF-Werten" werden auch in der Fluoreszenzmikroskopie eingesetzt. Beim MESF-Klassifizierungssystem, das ein relatives Kalibriersystem darstellt, werden die relativen Fluoreszenzintensitäten der jeweils mit Fluorophoren dotierten Teilchen - beispielsweise gekapselte bzw. in der Matrix der Teilchen eingeschlossene Fluorophore - oder Teilchen mit an der Teilchenoberfläche gebundenen vorliegenden Fluorophoren - beispielsweise Teilchen mit kovalent, konjugiert oder assoziativ an der Teilchenoberfläche gebundenem Fluorophor - auf die für den gleichen Fluorophor in einer Lösung bekannter Konzentration resultierenden Fluoreszenzintensitäten der Kalibrierbeads bezogen.

Eine direkte Messung der pro Fluorophor-Molekül oder für eine bestimmte Fluorophorkonzentration im Teilchen resultierenden Fluoreszenz wird dadurch verhindert, dass sich - bedingt durch die Umgebungssensitivität der Absorptions- und der Fluoreszenzeigenschaften des Fluorophors, sowie in Abhängigkeit von der Farbstoffbeladungskonzentration und den Eigenschaften des Farbstoffes selbst (d.h. dessen Tendenz zur Ausbildung molekularer Aggregate und

2

Assoziate (Aggregationstendenz), der jeweiligen spektralen Überlappung von Absorptions- und Emissionswellenlängen in der jeweiligen Matrix) auch durch Farbstoff-Farbstoff-Wechselwirkungen die signalbestimmenden spektroskopischen Eigenschaften, wie beispielsweise der Extinktionskoeffizient bei der jeweiligen Anregungswellenlänge - die jeweilige spektrale Lage der Emissionsbande oder die Fluoreszenzquantenausbeute der Farbstoffe beim Einbau oder Anbinden in den Teilchen ändern.

Der matrix- (Brechungsindexunterschied Lösungsmittel der Suspension - Matrix des Teilchens) und größenabhängige Beitrag der Streuung zum Absorptionssignal wird bei den angeführten Verfahren nicht erfasst. Andererseits wird die Kalibrierung eines Durchflusszytometers mit an sich leicht reproduzierbar herstellbaren Farbstofflösungen dadurch verhindert, dass ein Durchflusszytometer nur größere Objekte von mindestens mehreren 10 Nanometern Durchmesser, eher mehr, nicht jedoch einzelne Farbstoffmoleküle vermessen kann.

Die mit dem Durchflusszytometer i.a. integral gemessenen Signalintensitäten der Proben werden daher relativ zu Kalibrierbeads bestimmt; diese Signalintensitäten enthalten typischerweise auch noch wellenlängenabhängige Beiträge von gerätespezifischen optischen und opto-elektronischen Komponenten. Typischerweise stellen die der Durchflusszytometrie zugänglichen Proben Fluorophor-markierte Zellen oder im Falle von Fluoreszenzassays, oberflächenfunktionalisierte Teilchen mit gebundenem Analyten dar.

Dabei müssen aber für jedes Kalibrierbead-Fluorophor-System auch unter Berücksichtigung der jeweils verwendeten Teilchen-Matrix und des jeweils verwendeten Lösungsmittels jeweils neue MESF-Werte bestimmt werden. Wie ersichtlich, ist das ein sehr aufwendiger Prozess, der zudem nur eine relative Intensitätsskala liefert. Das kann durch das hier vorgestellte direkte Messprinzip und das Vorliegen geeigneter Teilchenstandards mit auf physikalische / spektroskopische Messgrößen rückführbaren Eigenschaften vereinfacht werden.

Wie dargelegt, sind mit dem bisher eingesetzten MESF-System nur vergleichende Aussagen möglich für ein und denselben Farbstoff, und dies oftmals auch nur mit einer relativ großen Messunsicherheit, da Änderungen der signalrelevanten optischen Eigenschaften des Fluorophors im Teilchen im Vergleich zur Lösung nicht erfasst werden. Dies betrifft insbesondere den molekularen (dekadischen) Extinktionskoeffizienten bei der jeweils verwendeten Anregungswellenlänge der eingebauten oder gebundenen Fluorophore und den Wert der Fluoreszenzquantenausbeute. Außerdem erfolgen vielfach nur integrale Fluoreszenzmessungen, und wellenlängenabhängige Beiträge von gerätespezifischen optischen und opto-elektronischen Komponenten werden nicht berücksichtigt bzw. korrigiert.

Eine echte Zuordnung von Helligkeitswerten zu gekapselten Fluorophoren (und natürlich auch zu gebundenen vorliegenden Fluorophoren) und die Übertragung dieser Helligkeitswerte auf Kalibrierwerkzeuge, die aus solchen Systemen aufgebaut wurden bzw. diese enthalten (z.B. Beads in Lösung und Beads, die eingebaut in festen Matrices vorliegen, Bead-basierte Phantome für bildgebende optische Verfahren, 2D- oder 3D-Systeme aus Beads in einer Matrix, Bead-Arrays, Bead-basierte Schichtsysteme, etc.) oder wo diese Beads als Sonden bzw. als Marker eingesetzt werden (z.B. Verwendung solcher Teilchen oder von andersförmigen Standards für die bessere Vergleichbarkeit von Untersuchungen am Gewebe in der optischen Bildgebung) oder als Analyseplattformen oder für andere optische Verfahren, die mit solchen Systemen arbeiten und Quantifizierungen erfordern, ist so bisher nicht möglich.

Andere bisherige Ansätze zur Bestimmung von Helligkeitswerten an Teilchen gehen von der Annahme aus, dass sich die Helligkeit als Produkt der Quantenausbeute des Fluorophors und seines molaren Extinktionskoeffizienten in der Matrix und der mittleren Fluorophorzahl pro Teilchen ergibt. Dabei werden weder Abschirm- noch Verstärkungseffekte erfasst, so dass diese Ansätze (abhängig vom Brechungsindex der Teilchenmatrix, des Lösungsmittels und der Teilchengröße) ungeeignet sind.

Diese Ansätze sind jedoch selbst für Teilchen, die lediglich wenige Nanometer groß sind, nur bedingt richtig. Insbesondere wird der Einfluss von Feldverstärkungseffekten, die sowohl von der jeweiligen Teilchengröße als auch von der jeweiligen Teilchenmatrix abhängen, sowie der Einfluss von konzentrations- und matrixabhängigen Farbstoff-Farbstoff-Wechselwirkungen bisher außer Acht gelassen und vernachlässigt.

Vor diesem Hintergrund besteht Bedarf an fluoreszenten Teilchen, mit bekannten, auf physikalische Messgrößen rückführbaren Helligkeiten, die als Standard verwendet werden können. Außerdem besteht Bedarf an einem Verfahren zur Ermittlung der absoluten Helligkeit der fluoreszenten Teilchen, um einen Vergleich zu ermöglichen.

In diesem Zusammenhang wird gemäß Anspruch 1 ein Verfahren vorgeschlagen, bei dem Helligkeit und damit die Lumineszenzintensität eines Teilchens auf grundlegende physikalische Parameter, wie die Quantenausbeute und den Absorptionswirkungsquerschnitt, zurückgeführt werden. Weitere Ausführungsformen, Modifikationen und Verbesserungen ergeben sich anhand der folgenden Beschreibung und der beigefügten Ansprüchen.

Erfindungsgemäß wird ein Verfahren zur Bestimmung der absoluten oder relativen Helligkeit eines lumineszenten Teilchens vorgeschlagen. Das Verfahren umfasst das Bereitstellen eines Probenvolumens, das ein Ensemble von lumineszenten Teilchen enthält, beispielsweise eine Teilchensuspension; das Ermitteln einer absoluten Photolumineszenzquantenausbeute der Teilchensuspension im Probenvolumen; das Ermitteln eines Absorptionswirkungsquerschnitts der Teilchen im Probenvolumen; und das Ermitteln der absoluten Helligkeit eines einzelnen Teilchens auf Basis der ermittelten absoluten Photolumineszenzquantenausbeute, des Absorptionswirkungsquerschnitts und Teilchenanzahl im Probenvolumen.

Das hier beschriebene Verfahren beruht auf der Messung an einem suspendierten Teilchenensemble, wobei dessen Photolumineszenzquantenausbeute und Absorptionswirkungsquerschnitt ermittelt wird. Auf Basis dieser Größen und der Kenntnis der Teilchenzahl, kann ein Helligkeitswert pro Teilchen ermittelt und jedem Teilchen zugeordnet werden. Die Helligkeit eines fluoreszenten Teilchens ist damit auf grundlegende physikalische und messtechnisch zugängliche Größen zurückgeführt.

Die Ermittlung des absoluten Helligkeitswerts pro Teilchen kann beispielsweise unter Verwendung der Strahlungstransportgleichung erfolgen, die geeignet gelöst wird. Dazu steht eine Vielzahl von geeigneten Ansätzen zur Verfügung, beispielsweise Monte-Carlo-Simulationen.

Erfindungsgemäß wird die absolute Photolumineszenzquantenausbeute und/oder der Absorptionswirkungsquerschnitt mittels wellenlängenaufgelöster Messung der Emission, Transmission und Reflektion der Teilchensuspensionen im Probenvolumen ermittelt. Dazu können die absolute Photolumineszenzquantenausbeute und/oder der Absorptionswirkungsquerschnitt gemäß einer Ausführungsform mittels einer Ulbrichtkugel gemessen werden. Dies kann wellenlängenabhängig erfolgen, z.B. mittels Ulbrichtkugelspektroskopie. Es ist auch möglich, andere alternative Verfahren zu verwenden. Beispielsweise kann die Messung der absoluten Photolumineszenzquantenausbeute und/oder des Absorptionswirkungsquerschnitts auch mittels Photoakustik erfolgen.

Gemäß einer Ausführungsform wird die absolute Helligkeit bei Anregungs- und Emissionswellenlängen im UV/vis/NIR - Bereich (250 bis 1500 nm) ermittelt. Die Anregung erfolgt insbesondere im Bereich von 300 nm bis 850 nm, kann aber auch längerwellig erfolgen. Die Detektion erfolgt insbesondere bei Emissionswellenlängen im vis/NIR Bereich von 350 bis 1000 nm, typischerweise im Bereich von 450 nm bis 850 nm, und insbesondere im Wellenlängenbereich von 500 bis 800 nm erfolgt. Es sind aber auch Messungen im NIR-Spektralbereich bis ca. 1600 nm denkbar, z.B. für Quantendot-beladene Teilchen.

Gemäß einer Ausführungsform wird zur Bestimmung der absoluten Helligkeit eines lumineszenten Teilchens ein Messaufbau bereitgestellt, der zur Bestimmung der Quantenausbeute und/oder des Absorptionskoeffizienten einer Suspension von lumineszenten Teilchen angepasst ist. Ein geeigneter Messplatzaufbau ist beispielsweise in EP 2 357 465A1 beschrieben, deren Offenbarungsinhalt bezüglich des Messplatzaufbaus hiermit vollständig aufgenommen wird.

Gemäß einer Ausführungsform wird der Absorptionskoeffizient $\mu_a$ der Teilchensuspension im Probenvolumen ermittelt.

Gemäß einer Ausführungsform erfolgt das Berechnen des Absorptionswirkungsquerschnitts $\sigma_a$ des lumineszenten Teilchens gemäß der Formel $\sigma_a = \dfrac{\overline{\mu_a}}{N}$, und wobei $\sigma_a$ - der Absorptionswirkungsquerschnitt des Teilchens [cm$^2$]; $\mu_a$ - der Absorptionskoeffizient [1/cm] und $N$ - die Zahl der Teilchen pro Volumeneinheit ist.

Erfindungsgemäß wird die absolute Helligkeit eines Teilchens als Produkt aus der gemessenen Quantenausbeute und dem gemessenen Absorptionswirkungsquerschnitt $\sigma_a$ [cm$^2$] eines Teilchens ermittelt, wobei das gebildete Produkt einem lumineszenten Teilchen zugeordnet wird.

Gemäß einer Ausführungsform wird die Größe und/oder die Größenverteilung der in der Suspension dispergiert vorliegenden lumineszenten Teilchen bestimmt.

Gemäß einer Ausführungsform erfolgt die Bestimmung der Größe bzw. der Größenverteilung mittels einer Messmethode ausgewählt unter: dynamischer Lichtstreuung (DLS); Elektronenmikroskopie, insbesondere Transmissionselektronenmikroskopie (TEM) und/oder Rasterelektronenmikroskopie (REM); Kleinwinkel-Röntgenbeugung (SAX); Zentrifugation, Ultrazentrifugation, optische Mikroskopie oder Laserbeugungs-Messung beziehungsweise Laserdiffraktometrie.

Gemäß einer Ausführungsform wird die Dielektrizitätskonstante des Materials der Teilchen und/oder die Dichte des Materials der Teilchen ermittelt.

Erfindungsgemäß sind die lumineszenten mono- oder polydispersen Teilchen im Größenbereich von 5 nm bis 5000 μm

Erfindungsgemäß wird die Verwendung zumindest eines lumineszenten Teilchens als ein Bezugsstandard oder als ein Markierungsreagenz für einen Teilchenbasierten Test vorgeschlagen, wobei dem Teilchen eine absolute Helligkeit zugeordnet wurde und wobei der Teilchenbasierte Test ausgewählt ist unter: Enzymimmunoassay (EIA), Enzyme-linked Immunoassay (ELISA); einem cytometrischen Analyseverfahren, insbesondere einem Analyseverfahren unter Verwendung eines Durchflusszytometers; einem Teilchenbasierten Test im Bereich der Umweltanalytik; einem Teilchenbasierten Tests im Bereich der Bioanalytik; einem Teilchenbasierten Test im Bereich der medizinischen Diagnostik; einem Teilchenbasierten Test im Bereich der pharmazeutischen Wirkstoffforschung; einem Teilchenbasierten Test zur Therapie-Kontrolle; einem Einsatz von Teilchen als Kontrastmittel; einem Einsatz von Quantenpunkt-Strukturen als Fluoreszenz-Label und/oder als Kontrastmittel; einem Einsatz von Phosphoren und Nanophosphoren oder/und dem Einsatz einer Teilchenemulsion.

Die vorstehend beschriebenen Ausführungsformen können beliebig miteinander kombiniert werden.

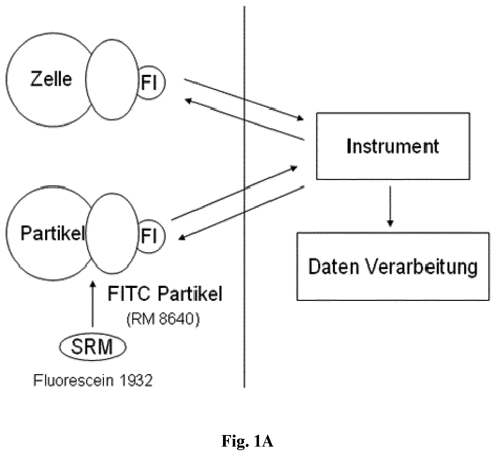

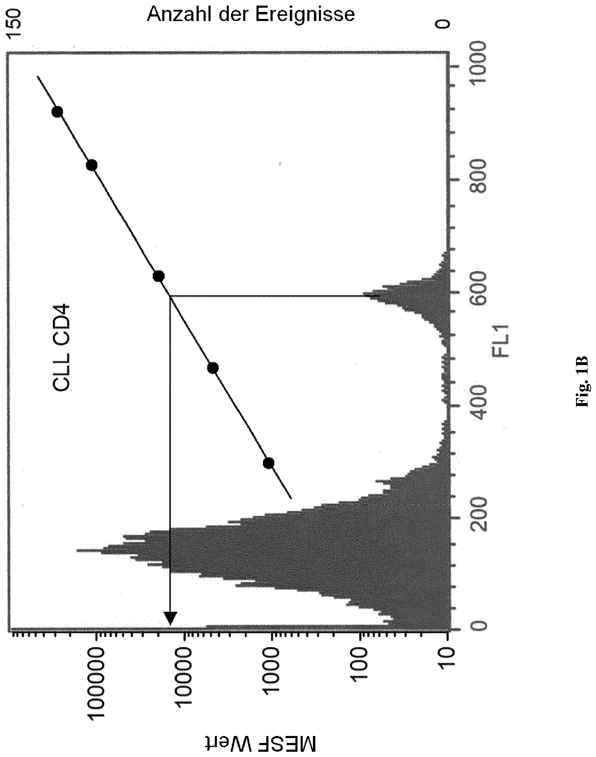

Die beiliegenden Figuren 1 bis 6 veranschaulichen Ausführungsformen und dienen zusammen mit der Beschreibung der Erläuterung der Prinzipien der Erfindung. Die Elemente der Figuren sind relativ zueinander und nicht notwendigerweise maßstabsgetreu. Gleiche Bezugszeichen bezeichnen entsprechend ähnliche Teile.

Fig. 1A zeigt die Beziehung der Fluoreszenzwerte von Kalibrierteilchen (Kalibrierpartikel) und real zu charakterisierendem Messobjekt (Zelle) am Beispiel des MESF-Verfahrens (measurable fluorescence of equivalent soluble fluorophore) für den am häufigsten in der Durchflusszytometrie verwendeten Fluorophor (hier als "Probe" bezeichnet) Fluoreszein, bzw. FITC.

Fig. 1B zeigt die Verwendung einer mit MESF-klassifizierten Beads erhaltenen Eichgeraden zur Kalibrierung der Intensitätsachse eines Durchflusszytometers.

Fig. 2 zeigt einen Ulbrichtkugelaufbau zur Bestimmung absoluter Quantenausbeuten, der für das hier vorgeschlagene Verfahren geeignet ist.

Fig. 3 zeigt absolute Quantenausbeuten von Teilchen unterschiedlicher Größe (Kurvenschar) und unterschiedlicher zur Markierung benutzter Fluorophore (linker und rechter Teil der Fig. 3).

Fig. 4 zeigt Möglichkeiten der unterschiedlichen Strahlführung im Integrationskugelaufbau zur Messung von totaler Transmission (a), diffuser Transmission (b) und diffuser Reflexion (c).

Fig. 5 zeigt den Absorptionswirkungsquerschnitt von 1$\mu$m Polystyrolteilchen (PSP), die mit unterschiedlichen Fluorophoren in unterschiedlicher molarer Konzentration beladen sind.

Fig. 6 zeigt die Helligkeitswerte von Teilchen unterschiedlicher Größe, die mit unterschiedlichen Farbstoffen beladen wurden.

Gemäß dem vorgeschlagenen Verfahren erfolgt die Bestimmung der Helligkeit pro Teilchen mit Ensemble-Messungen im UV/vis/NIR/IR-Spektralbereich an Teilchen in Suspensionen, an Teilchen, die in verschiedenen festen Matrices dispergiert vorliegen, und an Teilchen in Pulvern.

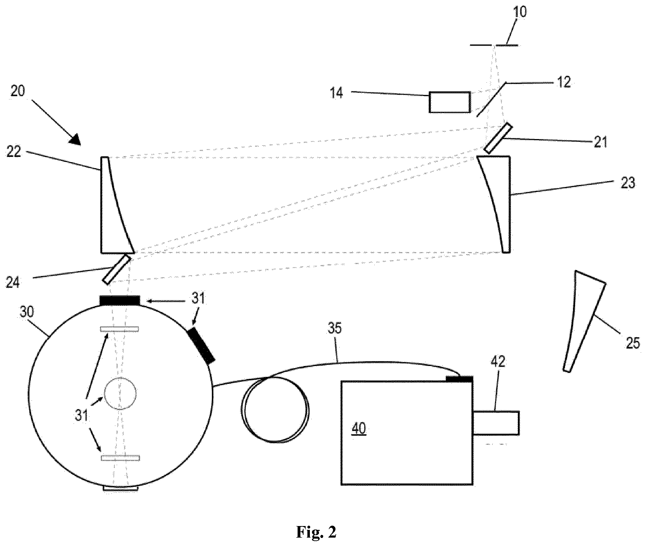

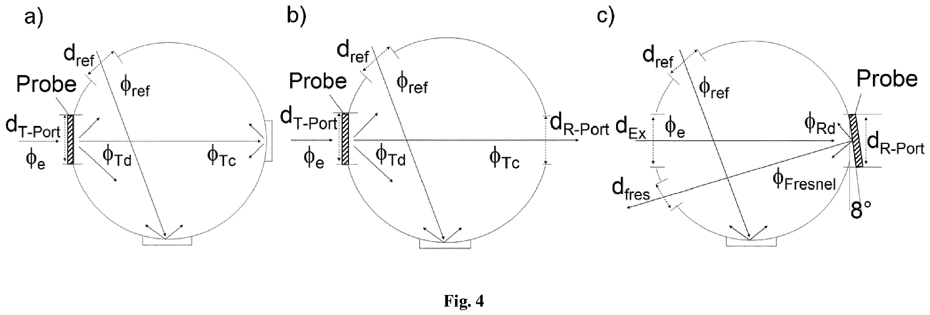

Dabei erfolgt die Bestimmung der Helligkeit pro Teilchen auf der Basis der absoluten Charakterisierung der Lumineszenzquantenausbeute bzw. Lumineszenz-Effizienz dieser Teilchen und der Bestimmung von Absorptionswirkungsquerschnitten pro Teilchen mit dem in Fig. 2 gezeigten Ulbrichtkugelmessplatz, wobei der Ulbrichtkugelmessplatz spektral aufgelöst die Emission, die Transmission und die Reflexion von transparenten und von streuenden Systemen messen kann (vgl. Fig. 4). Im Falle von Teilchensuspensionen ist prinzipiell auch eine relative Bestimmung der Quantenausbeute möglich als Basis für die Ermittlung der Helligkeitswerte mit der hier vorgestellten Methode, aber mit deutlich größeren Unsicherheiten verbunden, insbesondere bei zunehmender Streuung.

Zur Ermittlung der Helligkeitswerte werden als weitere Messgrößen die Teilchenzahl bzw. Teilchenkonzentration, die Teilchengröße und der Brechungsindex der Teilchen herangezogen.

Dabei wird die Quantenausbeute typischerweise absolut gemessen, der Absorptionswirkungsquerschnitt pro Teilchen wird dann aus den Ergebnissen von Reflexions-und Transmissionsmessungen unter Verwendung eines Reflexionsstandards berechnet. Als Reflexionsstandard dient ein Weiß-Standard mit bekannter Reflektivität. Die Teilchenzahl wiederum ergibt sich entweder rechnerisch aus der Mie-Theorie oder aus gravimetrischen Messungen, bzw. kann bei bekannter Teilchengröße und Materialdichte aus der jeweils zur Messung verwendeten Einwaage berechnet werden.

Die Teilchengröße kann auf verschiedene Art und Weise bestimmt werden. Beispielsweise an Hand von Messungen mittels DLS (Dynamischer Lichtstreuung), TEM (Transmissions-Elektronenmikroskopie), REM (Rasterelektronenmikroskopie), SAXS (Kleinwinkel-Röntgenbeugung), mittels Zentrifugation, Mikroskopie oder mittels Laserbeugungs-Messungen.

Für Teilchen mit Dimensionen im Bereich der Lichtwellenlänge kann die Größe der Teilchen prinzipiell aber ebenso mit Hilfe von Integrationskörper-Messungen, beispielsweise mit einer Integrationskugel-Messanordnung (Ulbrichtkugelmessung) erfolgen.

Der wellenlängenabhängige Brechungsindex ist für unterschiedliche praxisrelevante Matrices entweder aus Literaturdaten bekannt bzw. kann aus entsprechenden Datenbanken ermittelt werden.

Unter Berücksichtigung dieser entweder durch Messung und/oder Berechnung bzw. Tabellen zugänglichen Daten können erfindungsgemäß die an einer dispergierte oder solubilisierte Teilchen enthaltenden Probe gemessenen Helligkeitswerte auf grundlegende spektroskopische und physikalische Größen zurückgeführt werden. Daraus ergibt sich der grundlegende Vorteil, dass für alle Teilchengestützten Fluoreszenzverfahren eine rückführbare Intensitätsskala aufgebaut werden kann, die z.B. für die Rückführung von Fluoreszenz-Messungen bzw. Fluoreszenz-Intensitäten auf molekulare Größen und radiometrische Skalen geeignet ist.

Unter Fluoreszenzmessungen werden dabei insbesondere verstanden: 3D- und 2D-Systeme, alle Fluoreszenzmethoden, die Teilchen als Plattformen verwenden und/oder die für die Durchführung von Fluoreszenzassays eingesetzt werden, beginnend beispielsweise bei der Durchflusszytometrie, über die Fluoreszenzmikroskopie bis zur Mikrotiterplatten-basierten Fluorometrie, einschließlich der Mikroarray-Technologie, der Fluoreszenzsensorik sowie Teilchenbasierten bildgebenden Fluoreszenzverfahren (z.B. Fluoreszenztomographie, FRI), z.B. für Untersuchungen an Gewebe, Gewebeschnitten, in Zellsuspensionen, Ausstrichen oder in Gelen, beispielsweise in Agarose- oder Polyacrylamid-Gelschichten.

Es können so verschiedene Teilchen direkt miteinander verglichen werden sowie über bereits hinsichtlich ihrer Helligkeit charakterisierte Teilchen auch Helligkeitswerte zu anderen Fluoreszenzstandards zugeordnet werden, die auf diesen Teilchen basieren.

Häufig werden derartige Teilchen in den entsprechenden Tests als "beads" bezeichnet. Nachfolgend werden auch derartige beads unter dem oben erläuterten Begriff Teilchen verstanden, bzw. unter "beads" werden die vorgenannten Teilchen verstanden. Damit sollen die Begriffe bead/beads, und der Begriff Teilchen nachfolgend als äquivalente Begriffe behandelt werden.

Fig. 1A veranschaulicht das eingangs beschriebene MESF-Verfahren als Beispiel eines relativen Klassifizierungssystems von Fluoreszenzintensitäten von Bezugsfarbstoff (SRM), farbstoffmarkiertem Teilchen (Partikel) und Messobjekt Zelle am Beispiel von Fluoreszein (Probe), die eine bestimmte Fluoreszenzintensität (FI) aufweist. Insbesondere ist die schematische Zuordnung von Fluoreszenzintensitäten (FI) eines entsprechenden Standard-Referenz-Materials (SRM), umfassend den Fluorophor Fluoreszein gezeigt. Der Fluorophor Fluoreszein ist als Standard Reference Material (SRM) 1932 vom National Institute for Standards and Technology (NIST, USA) mit zertifizierter Reinheit verfügbar.

Aus dem Abgleich der jeweils gemessenen Fluoreszenzintensitäten wird eine relative Zuordnung verschiedener Intensitäten auf eine Bezugsskale erreicht. Das ist in Fig. 1B für eine beispielartige Anwendung zum Nachweis spezifisch fluoreszenzmarkierter Zellen gezeigt, die ein für chronisch-lymphozytische Leukämie (CLL) typisches Oberflächenantigen (CD4) tragen. Der am häufigsten in der Durchflusszytometrie verwendete Fluorophor Fluoreszein (FITC) lieferte die im Messkanal FL1 erfassten Fluoreszenzintensitäten, welche sich zwei deutlich erkennbaren Peaks zuordnen lassen.

Um eine interessierende Fluoreszenzintensität dem korrespondierenden MESF-Wert zuordnen zu können, wird die zuvor ermittelte Kalibriergerade verwendet. Das ist durch den auf die Ordinate weisenden Pfeil angedeutet. Auf diese Art und Weise kann die Intensitätsachse eines Durchflusszytometers mit MESF-klassifizierten Beads für die jeweils verwendeten Fluorophore kalibriert werden, wenn für den jeweiligen Fluorophor ein Standard-Referenz-Material vorliegt.

Fig. 2 zeigt einen für das hier vorgeschlagene Verfahren nutzbaren Ulbrichtkugelaufbau zur Bestimmung absoluter Quantenausbeuten. Das Licht eines zur Anregung genutzten Monochromators wird vom Ausgangsspalt 10 auf einen Strahlteiler 12 geleitet. Ein vorbestimmter Teil davon gelangt auf einen Referenz-Detektor14. Der andere Teil wird über die gezeigte Parabolspiegelanordnung 20 umfassend Parabolspiegel 22, 23 und Planspiegel 21, 24 in eine Integrationskugel 30 gelenkt, welche an geeigneten Positionen über Blenden 31 verfügt. Das von einer Sonde zu erfassende Messsignal wird anschließend über eine optische Faser 35 zu eine Spektrographen 40 geführt, dort spektroskopiert und mittels einer CCD-Kamera 42 erfasst. Parabolspiegel 25 dient einer indirekten Beleuchtung der Probe. Auch aus solch einer Messanordung kann die Quantenausbeute bestimmt werden. Im Falle der Reflektionsmessung dient diese Anordnung zum Ausgleich der veränderten Ulbrichtkugelreflektivität (Referenzmessung), siehe weiter unten und EP 2 357 465A1, deren Offenbarungsinhalt hiermit vollständig aufgenommen wird.

Mittels dieses Ulbrichtkugelaufbaus werden die absoluten Lumineszenzquantenausbeuten und/oder Helligkeiten des interessierenden Teilchensystems bestimmt sowie Reflexions- und Transmissionsmessungen durchgeführt. Die Lösung der Strahlungstransportgleichung liefert Absorptions- und Streukoeffizienten der Teilchen-Probe. Mittels der allgemeinen Streutheorie nach Mie können bei geeigneten Voraussetzungen die grundlegenden Materialparameter wie der Brechungsindex oder die Partikelanzahl und -größe für die Bestimmung des und Absorptionswirkungsquerschnittes berechnet werden.

Unter Verwendung des für die jeweilige Teilchenmatrix spezifischen Brechungsindex kann ebenso ein Polydispersitäts-Index der Probe ermittelt werden. Bei Kenntnis des Polydispersitätsindex können angenäherte Wirkungsquerschnitte der Teilchen für Streuung und Absorption berechnet werden, so dass die Anwendung des hier vorgeschlagenen Verfahrens nicht allein auf monodisperse Teilchenproben beschränkt ist. Ebenso können schmalbandige Teilchengrößenverteilungen oder polydisperse Teilchenproben charakterisiert werden. Das Verfahren ist ebenso nicht allein auf sphärische Teilchen beschränkt, sondern ebenso auf Dispersionen von Stäbchen (rods), oder Dispersionen anderer Teilchenformen, beispielsweise auf Dispersionen von Polyedern im Nano bis Mikrometerbereich anwendbar.

Die Quantenausbeute stellt das Verhältnis der Zahl emittierter Photonen zur Zahl der absorbierten Photonen dar. Die sogenannte Helligkeit (bzw. brightness) von Fluorophoren wurde bisher durch das Produkt aus absoluter Quantenausbeute $\Phi_f$ und molekularem dekadischen Absorptionskoeffizienten $\varepsilon$ [L/(mol cm)] beschrieben.

Die Transmission einer Lösung von Farbstoffmolekülen folgt dem Lambert-Beer'schen Gesetz:

$$T_F = \frac{I}{I_0} = e^{-\alpha d} = e^{-N_0 \sigma_a l} = e^{-N_A \sigma_a c l} \qquad \text{(Gl. 0)},$$

wobei $I_0$ -die Lichtintensität beim Eintritt in die Probe, I - die Intensität des Lichtes, das aus der Probe austritt, $T_F$ - die Transmission der Farbstofflösung, $N_0$ - die Molekülteilchendichte, $\alpha$ - den molaren Absorptionskoeffizienten, $N_A$ - die Avogadro'sche Zahl, c - die molare Konzentration des Farbstoffs (Fluorophors), l - die Dicke der vermessenen Probenschicht und $\sigma_a$ - den Absorptionswirkungsquerschnitt darstellt. Der Absorptionswirkungsquerschnitt stellt eine hypothetische Fläche dar, mit der das Farbstoffmolekül ein Photon absorbiert.

Im Gegensatz zu den üblicherweise ausschließlich auf molekulare Größenskalen angewandten Theorien und Modellen schlagen wir vor, die Helligkeitswerte von Teilchen durch das Produkt des Absorptionswirkungsquerschnittes eines Teilchens $\sigma_a$ [cm$^2$] und der an einer Teilchendispersion gemessenen Quantenausbeute $\Phi_f$ auszudrücken. Das eröffnet eine unserer Kenntnis nach völlig neue Möglichkeit zur Beschreibung und damit zur Normierung von lumineszierenden Teilchen. Anstelle des Absorptionswirkungsquerschnittes kann gemäß Hulst, H.C. van d. (Light scattering by small particles. Dover Publications, 1981 ISBN 0-486-64228-3) auch die Absorptionseffizienz $Q_{abs}$ verwendet werden. Gemäß dieser Publikation (vgl. Seiten 13 und 14) ist die Absorptionseffizienz $Q_{abs}$ der Quotient aus Absorptionswirkungsquerschnitt und geometrischem Querschnitt $G = \pi a^2$, wobei a der Radius des Partikels ist. Deren Verwendung erweitert zusätzlich die Anwendbarkeit des von uns vorgeschlagenen Verfahrens zur Bestimmung der Helligkeit eines Teilchens und gestattet eine Normierung.

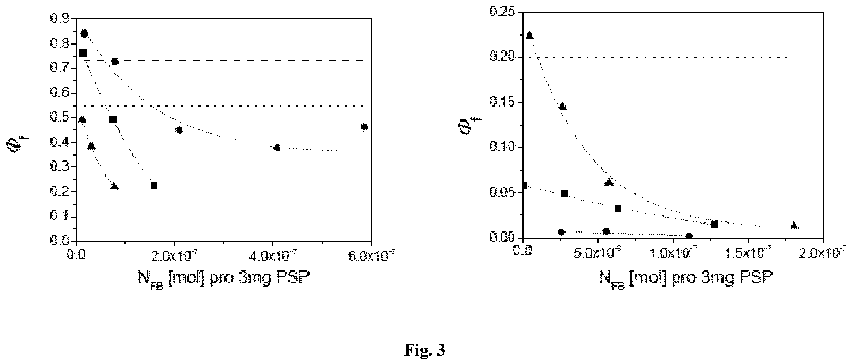

Durch die vorgeschlagene Normierung wird eine Vergleichbarkeit unterschiedlicher Teilchengrößen erreicht. Das ist beispielhaft in Fig. 3 gezeigt. Insbesondere zeigt der linke Teil der Fig. 3 Messwerte der absoluten Quantenausbeute von Teilchen unterschiedlicher Größe, die mit dem Fluorophor Nilrot beladen sind. Der rechte Teil der Fig. 3 zeigt Messwerte der absoluten Quantenausbeute von Teilchen jeweils unterschiedlicher Größe, die mit dem Fluorophor F006 beladen sind. Dabei steht $N_{FB}$ - für die absolute Quantenausbeute, angegeben in mol pro 3 mg Polystyrol-Teilchen (PSP).

Damit stellt die Kombination der absoluten Photolumineszenzquantenausbeute mit den experimentell ermittelten Absorptionseffizienzen bzw. Absorptionswirkungsquerschnitten pro Teilchen zu einem Teilchen-Effizienzfaktor bzw. zu einem Helligkeitswert pro Teilchen einen wesentlichen Bestandteil des hier beschriebenen Verfahrens dar.

Insbesondere kann die Quantenausbeute $\Phi_f$ streuender Medien, beispielsweise einer Lösung mit darin suspendiert vorliegenden fluoreszenzmarkierten Teilchenensembles, oder einer Oberfläche, welche lumineszierende Teilchen trägt, oder auch eine transparente Schicht, in welcher lumineszente Teilchen eingebettet vorliegen, unter Verwendung eines Ulbrichtkugel-Messplatzes gemäß der Fign. 2 und 4 oder auch mittels der photoakustischen Spektroskopie bestimmt werden und ergibt sich als:

$$\Phi_f = \frac{N_{em}}{N_{abs}} \qquad \text{(Gl. 1)},$$

wobei $N_{em}$ - für die Zahl der emittierenden Teilchen und $N_{abs}$ - für die Zahl der absorbierenden Teilchen steht. Ebenso können Absorptionskoeffizienten der vorstehend beschriebenen Proben unter Verwendung der Ulbrichtkugel- Spektroskopie bestimmt werden. Bei einem geeigneten Aufbau, beispielsweise dem in den Fign. 2 und 4 gezeigten, lassen sich Quantenausbeute und Absorptionskoeffizienten mit dem gleichen Aufbau bestimmen.

Beispielsweise können aus Reflexions- und Transmissionsmessungen gemäß der in Fig. 4 gezeigten Strahlführung die Absorptionskoeffizienten $\mu_a$ einer Probe mit z.B: einer inversen Monte Carlo Simulation oder mittels anderer Verfahren zur Lösung der Strahlungstransportgleichung bestimmt werden.

Mit Kenntnis der Teilchenanzahl N, die mit Kenntnis der Teilchengröße durch z.B. Wiegen eines aliquotierten Teils einer Probe bestimmt werden kann, wird der Absorptionswirkungsquerschnitt $\sigma_a$ eines einzelnen Teilchens berechnet aus:

$$\sigma_a = \frac{\mu_a}{N} \qquad \text{(Gl.2)}$$

wobei $\mu_a$ - der Absorptionskoeffizient des Teilchenensembles und N - die Teilchenzahl ist.

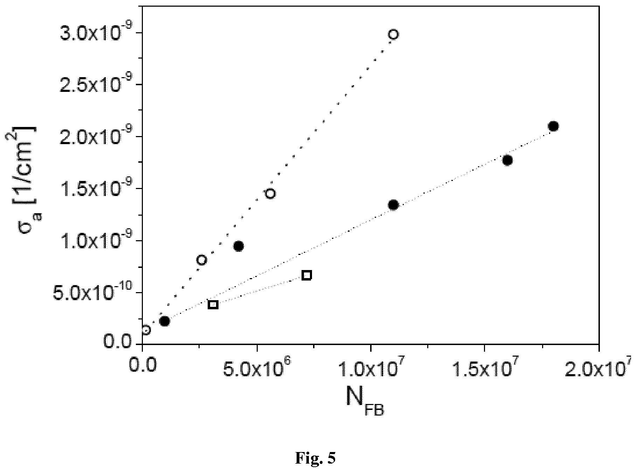

In Fig. 5 ist der Absorptionswirkungsquerschnitt von 1 $\mu$m Polystyrolteilchen, die mit den Fluorophoren Nilrot (offene Kreise), F006 (volle Kreise) und C153 (offene Quadrate) beladen sind, aufgetragen über die in den Teilchenn eingebaute Farbstoffmenge. Dabei bedeutet $N_{FB}$ - absolute Quantenausbeute, angegeben in [mol pro 3 mg PSP], wobei PSP für Polystyren- bzw. für Polystyrol-Teilchen steht.

Ist die Teilchengröße unbekannt, so kann sie beispielsweise mittels optischer Verfahren (Laserbeugung, DLS), mittels (Ultra-)Zentrifugation oder beispielsweise mittels Elektronenmikroskopie oder mit Hilfe elektronemikroskopischer Techniken bestimmt werden.

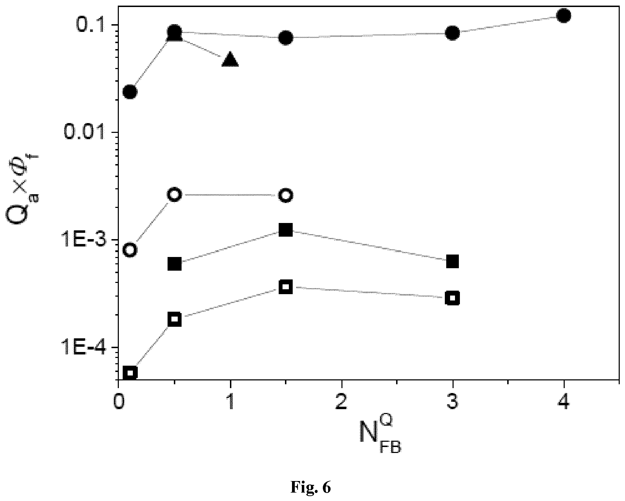

Fig. 6 zeigt die Helligkeitswerte von Teilchenn unterschiedlicher Größe, die mit unterschiedlichen Farbstoffen beladen wurden. Die Helligkeitswerte sind jeweils aufgetragen über der für die Herstellung verwendeten Farbstoffmenge, angegeben in mmol. Die Teilchendurchmesser betrugen $1\mu m$ (volle Symbole) und 100 nm (leere Symbole). Die verwendeten Fluorophore sind Nilrot (Kreise), F006 (Quadrate), C153 (Dreiecke).

Während - wie eingangs dargelegt - auch Quantenausbeuten bisher zumeist relativ zu einem Standard ermittelt wurden, was naturgemäß zu hohen Unsicherheiten führt und überhaupt nur für transparente Systeme machbar ist, stellt das hier vorgeschlagene und beschriebene Verfahren ein von diesen Unsicherheiten weitestgehend freies Verfahren auch für nicht transparente Teilchen-Systeme dar.

Mit dem vorstehend beschriebenen Verfahren wird somit eine allgemeine Methode zur Bestimmung von Helligkeitswerten pro Teilchen bereitgestellt, die auf Ensemble-Messungen basiert, z.B. auf Messungen an Teilchensuspensionen, auf Messungen an Teilchen-dekorierten Oberflächen, Teilchendispersionen und als Pulver vorliegenden Teilchen auf der Basis der absoluten Charakterisierung der Lumineszenz-Effizienz von Teilchen im UV/vis/NIR-Spektralbereich, insbesondere im Spektralbereich von 300 nm bis 1000 nm.

Die Bestimmung der Absorptionswirkungsquerschnitte pro Teilchen erfolgt entweder optisch mit einem Messplatz, der spektral aufgelöst Emission, Transmission und Reflexion von transparenten und/oder streuenden Systemen messen kann, beispielsweise mit einem geeignet angepassten Integrationskörper, beispielsweise einem Ulbrichtkugelmessplatz gemäß EP 2 357 465A1. Alternativ kann die Bestimmung der Absorptionswirkungsquerschnitte pro Teilchen z.B. mittels photo-akustischer Messungen vorgenommen werden.

Es wird weiterhin eine allgemeine Methode zur Charakterisierung und Klassifizierung von Teilchen bereitgestellt, die sich z.B. für die Charakterisierung von Teilchen im Bereich der Umwelt- und Bioanalytik, in der medizinischen Diagnostik etc. und für alle Methoden, die Teilchen-basierte Assays und Arrays einsetzen, eignet.

Damit lässt sich ein einfaches, auf grundlegende spektroskopische und physikalische Messgrößen rückführbares Referenzsystem für Fluoreszenz-Messungen aufbauen. Das hier vorgeschlagene Referenzsystem ist z.B. für die Standardisierung von Fluoreszenz-Messungen und für deren verbesserte und vor allem für eine universell vergleichbare Quantifizierung geeignet.

Dieser Ansatz ist prinzipiell für alle Teilchensysteme geeignet, unabhängig von ihrer Größe und geometrischen Form (z.B. auch andere definierte geometrische Formen, wie Stäbchen oder Dispersionen oder Suspensionen von nano- oder mikroskaligen Polyedern). Dieser Ansatz ist auch zur Charakterisierung von Teilchen geeignet, deren Helligkeitswerte abhängig sind von der sie umgebenden Matrix, nur ist dann die einfache Transferierung dieser Angaben auf andere Matrices oder andere, aus diesen Teilchen aufgebaute Systeme nicht mehr direkt möglich. Dem Fachmann sind die Methoden der Übertragung der vorliegenden Angaben auf andere Materialkombinationen - beispielsweise unter Verwendung der Brechungsindices dieser Materialien - bekannt.

Das vorgeschlagene Verfahren entspricht weitestgehend den in einer realen Mess-Situation anzutreffenden Bedingungen und ist auf nahezu jegliche Teilchenmaterialien bzw. transparente oder streuende und absorbierende und nicht absorbierende Matrices anwendbar.

Die erhaltenen Aussagen sind weiterhin unabhängig von der Form und der Größe der Teilchen im bezeichneten Bereich, wobei der bevorzugte Größenbereich der Teilchen von der Nanometer-Skala bis in den Mikrometer-Bereich reicht insbesondere bevorzugt von 5 nm bis $5000\mu m$. Die Teilchen (beads) können als Marker mit Größen von ca. 2 nm bis mehrere 100 nm, als Analysenplattformen i.a. 1000 nm bis etliche 1000 nm (Durchflusszytometrie z.B. 1000 nm bis max 10.000 nm, eher 5000 nm) verwendet werden.

Ein weiterer Vorteil des vorgeschlagenen Verfahrens besteht darin, dass die Bestimmung von quasi-absoluten Helligkeitswerten ermöglicht wird, die eine normierte einheitslose Größe darstellt. So wird eine Vergleichbarkeit unterschiedlichster Teilchensorten unabhängig von ihren Materialeigenschaften und Größe erreicht.

Über die Annahme, dass sich durch die Kapselung der Fluorophore im Teilchen die optischen Eigenschaften des Fluorophor-Teilchen-Systems nicht ändern, können diese Helligkeitswerte prinzipiell auf Systeme übertragen werden, die solche Teilchen enthalten bzw. die aus diesen Teilchen aufgebaut sind oder aus derartigen lumineszierenden Teilchen aufgebaut werden können. Voraussetzung dafür sind u.a. die Abwesenheit von Teilchen-Teilchen-Wechselwirkungen sowie geeignete geometrische Strukturen. Auf jeden Fall ist eine direkte Rückführung auf die Teilchensysteme möglich. Mit Kenntnis der Helligkeitswerte können prinzipiell die optischen Eigenschaften von Systemen mittels mathematischer Methoden vorausgesagt werden, die diese Teilchen enthalten bzw. die aus diesen Teilchen aufgebaut sind oder aus derartigen lumineszierenden Teilchen aufgebaut werden können.

Mit Bezug zur Standardisierung derartiger Teilchen, zur Standardisierung Teilchenbasierter Analyse- und Nachweisverfahren sowie zur Standardisierung der zur Durchführung lumineszenzoptischer Messungen verwendeten optischen Messgeräte stellt das vorgeschlagene Verfahren eine Methode zur Rückführung der jeweils erhobenen Daten auf eine absolute und auf grundlegende spektroskopische und physikalische Größen gestützte Helligkeits-Skala zur Verfügung.

Während der Einfluss von Feldverstärkungseffekten, die sowohl von der jeweiligen Teilchengröße als auch von der Teilchenmatrix abhängen, sowie der Einfluss von konzentrations- und matrixabhängigen Farbstoff-Farbstoff-Wechselwirkungen bisher völlig außer Acht gelassen und vernachlässigt wurde, liefert die hiermit vorgeschlagenen Methode verlässliche und universell einsetzbare Bezugswerte.

Vor diesem Hintergrund bietet das vorgeschlagene Verfahren eine physikalisch begündete und verlässliche Möglichkeit der Rückführung von Lumineszenz-Daten von großer praktischer Bedeutung und universeller Nutzbarkeit.

Wenngleich hierin spezifische Ausführungsformen dargestellt und beschrieben worden sind, liegt es im Rahmen der vorliegenden Erfindung, die gezeigten Ausführungsformen geeignet zu modifizieren, ohne vom Schutzbereich der vorliegenden Erfindung abzuweichen, welcher in den nachfolgenden Ansprüchen definiert ist.

**Patentansprüche**

1. Verfahren zur Bestimmung einer absoluten Helligkeit eines lumineszenten Teilchens, umfassend:

   - Bereitstellen eines Probenvolumens, das ein Ensemble von lumineszenten Teilchen enthält;
   - Ermitteln einer absoluten oder einer relativen Photolumineszenzquantenausbeute des Teilchenensembles im Probenvolumen;
   - Ermitteln eines Absorptionswirkungsquerschnitts oder der Absorptionseffizienz der Teilchen im Probenvolumen;
   - Ermitteln der absoluten Helligkeit eines einzelnen Teilchens auf Basis der ermittelten absoluten Photolumineszenzquantenausbeute, des Absorptionswirkungsquerschnitts oder der Absorptionseffizienz, und einer Teilchenanzahl im Probenvolumen, wobei die absolute Helligkeit durch ein Produkt des Absorptionswirkungsquerschnitts eines Teilchens $\sigma_a$ [cm$^2$] und der an einer Teilchendispersion gemessenen Quantenausbeute $\Phi_f$ ausgedrückt wird;
   wobei die lumineszenten Teilchen ausgewählt sind unter im Probenvolumen suspendierten oder dispergierten Teilchen im Größenbereich von 5 nm bis 5000 $\mu$m, und wobei die absolute Photolumineszenzquantenausbeute und/oder der Absorptionswirkungsquerschnitt bestimmt wird mittels einer wellenlängenaufgelösten Messung einer Emission, einer Transmission und einer Reflektion.

2. Verfahren nach Anspruch 1, wobei zum Ermitteln der absoluten Photolumineszenzquantenausbeute und/oder des Absorptionswirkungsquerschnitts eine Ulbrichtkugel verwendet wird.

3. Verfahren nach Anspruch 1, wobei das Ermitteln der absoluten Photolumineszenzquantenausbeute und/oder des Absorptionswirkungsquerschnitts mittels Photoakustik erfolgt.

4. Verfahren nach einem der Ansprüche 1 bis 3, wobei das Ermitteln der relativen Photolumineszenzquantenausbeute und/oder des Absorptionswirkungsquerschnitts relativ zu einem Standard erfolgt.

5. Verfahren nach einem der Ansprüche 1 bis 4, wobei das Ermitteln der Photolumineszenzquantenausbeute und/oder des Absorptionswirkungsquerschnitts bei Anregungswellenlängen im UV-, vis- oder NIR-Bereich, insbesondere im Bereich von 250 bis 1500 nm, insbesondere im Bereich von 300 nm bis 850 nm erfolgt.

6. Verfahren nach einem der Ansprüche 1 bis 5, wobei das Ermitteln der Photolumineszenzquantenausbeute und/oder des Absorptionswirkungsquerschnitts bei Emissionswellenlängen im vis- oder NIR-Bereich von 400 bis 1000 nm, typischerweise im Bereich von 300 nm bis 1600 nm, insbesondere im Wellenlängenbereich von 350 bis 1000 nm erfolgt.

7. Verfahren nach einem der Ansprüche 1 bis 6, wobei ein Absorptionswirkungsquerschnitt $\sigma_a$ eines lumineszenten Teilchens gemäß der Formel

$$\sigma_a = \frac{\mu_a}{N} \text{ ermittelt wird, wobei}$$

   $\sigma_a$ - der Absorptionswirkungsquerschnitt des Teilchens [cm$^2$];
   $\mu_a$ - der Absorptionskoeffizient [1/cm] des Teilchenensembles im Probenvolumen; und
   $N$ - die Zahl der Teilchen pro Volumeneinheit ist, wobei

der Absorptionskoeffizient $\mu_a$ des Teilchenensembles auf Basis der Emission, der Transmission und der Reflektion des Teilchenensembles im Probenvolumen ermittelt wird.

8. Verfahren nach einem der Ansprüche 1 bis 7, wobei die absolute Helligkeit eines einzelnen Teilchens als Produkt aus Photolumineszenzquantenausbeute und Absorptionswirkungsquerschnitt $\sigma_a$ [cm$^2$] eines Teilchens definiert wird, und wobei die absolute Helligkeit einem lumineszenten Teilchen oder einer Vielzahl der lumineszenten Teilchen zugeordnet wird.

9. Verfahren nach einem der Ansprüche 1 bis 8, weiterhin umfassend

    - Bestimmen einer Größe und/oder einer Größenverteilung der in der Suspension dispergiert vorliegenden lumineszenten Teilchen.

10. Verfahren nach Anspruch 9, wobei das Bestimmen durchgeführt wird mit einer Messmethode ausgewählt unter: dynamischer Lichtstreuung (DLS); Elektronenmikroskopie, insbesondere Transmissionselektronenmikroskopie (TEM) und/oder Rasterelektronenmikroskopie (REM); Kleinwinkel-Röntgenbeugung (SAX); Ultrazentrifugation oder Laserbeugungs-Messung beziehungsweise Laserdiffraktometrie.

11. Verfahren nach einem der Ansprüche 1 bis 10, weiterhin umfassend

    - Ermitteln einer Dielektrizitätskonstante des Materials der Teilchen und/oder Ermitteln einer Dichte des Materials der Teilchen.

12. Verfahren nach einem der Ansprüche 1 bis 11, wobei das Teilchenensemble eine Teilchensuspension ist, welche das Probenvolumen füllt.

13. Verwendung zumindest eines lumineszenten Teilchens als ein Bezugsstandard oder als ein Nachweisreagenz für einen Teilchenbasierten Test, wobei dem lumineszenten Teilchen eine absolute Helligkeit zugeordnet ist, die gemäß dem Verfahren nach einem der Ansprüche 1 bis 12 bestimmt wird, und wobei der Teilchenbasierte Test ausgewählt ist unter: Enzymimmunoassay (EIA), Enzyme-linked Immunoassay (ELISA); einem cytometrischen Analyseverfahren; einem durchflusszytometrischen Analyseverfahren; einem Teilchenbasierten Test im Bereich der Umweltanalytik; einem Teilchenbasierten Tests im Bereich der Bioanalytik; einem Teilchenbasierten Test im Bereich der medizinischen Diagnostik; einem Teilchenbasierten Test im Bereich der pharmazeutischen Wirkstoffforschung; einem Teilchenbasierten Test zur Therapie-Kontrolle; einem Einsatz von Teilchen als Kontrastmittel; einem Einsatz von Quantenpunkt-Strukturen als Fluoreszenz-Label und/oder als Kontrastmittel; einem Einsatz von Phosphoren und Nanophosphoren oder/und dem Einsatz einer Teilchenemulsion.

14. Verfahren zur Herstellung eines Standardsatzes lumineszenter Teilchen, wobei lumineszente Teilchen bereitgestellt werden, die absolute Helligkeit der lumineszenten Teilchen gemäß dem Verfahren nach einem der Ansprüche 1 bis 12 ermittelt wird, und den Teilchen die so ermittelte absolute Helligkeit zugewiesen wird.

15. Verfahren nach Anspruch 14, wobei ein Satz unterschiedlicher Teilchen bereitgestellt wird, wobei sich sie die Teilchen im Fluorophor und/oder der Konzentration des Fluorophors und/oder in der Teilchengröße unterscheiden.

**Claims**

1. A method for determining an absolute brightness of a luminescent particle, comprising:

    - providing a sample volume containing an ensemble of luminescent particles;
    - determining an absolute or a relative photoluminescence quantum yield of the particle ensemble in the sample volume;
    - determining an absorption cross-section or the absorption efficiency of the particles in the sample volume;
    - determining the absolute brightness of an individual particle based on the determined absolute photoluminescence quantum yield, the absorption cross-section or the absorption efficiency and a particle count in the sample volume, the absolute brightness being expressed by a product of the absorption cross-section of a particle $\sigma_a$ [cm$^2$] and the quantum yield $\Phi_f$ measured in a particle dispersion,
    the luminescent particles being selected from particles in the size range of 5 nm to 5000 $\mu$m suspended or

dispersed in the sample volume, and the absolute photoluminescence quantum yield and/or the absorption cross-section being determined by way of a wavelength-resolved measurement of an emission, a transmission and a reflection.

2. The method according to claim 1, wherein an integrating sphere is used to determine the absolute photoluminescence quantum yield and/or the absorption cross-section.

3. The method according to claim 1, wherein the absolute photoluminescence quantum yield and/or the absorption cross-section are determined by way of photoacoustics.

4. The method according to any one of claims 1 to 3, wherein the relative photoluminescence quantum yield and/or the absorption cross-section are determined relative to a standard.

5. The method according to any of one of claims 1 to 4, wherein the photoluminescence quantum yield and/or the absorption cross-section are determined at excitation wavelengths in the UV, vis or NIR range, in particular in the range of 250 to 1500 nm, and in particular in the range of 300 nm to 850 nm.

6. The method according to any of one of claims 1 to 5, wherein the photoluminescence quantum yield and/or the absorption cross-section are determined at emission wavelengths in the vis or NIR range of 400 to 1000 nm, typically in the range of 300 nm to 1600 nm, and in particular in the wavelength range of 350 to 1000 nm.

7. The method according to any one of claims 1 to 6, wherein an absorption cross-section $\sigma_a$ of a luminescent particle is determined according to formula

$$\sigma_a = \frac{\mu_a}{N}\text{, wherein}$$

   $\sigma_a$ is the absorption cross-section of the particle [cm$^2$];
   $\mu_a$ is the absorption coefficient [1/cm] of the particle ensemble in the sample volume; and
   N is the number of particles per unit volume,

   the absorption coefficient $\mu_a$ of the particle ensemble being determined based on the emission, the transmission and the reflection of the particle ensemble in the sample volume.

8. The method according to any one of claims 1 to 7, wherein the absolute brightness of an individual particle is defined as the product of the photoluminescence quantum yield and the absorption cross-section $\sigma_a$ [cm$^2$], and the absolute brightness is associated with a luminescent particle or a plurality of the luminescent particles.

9. The method according to any one of claims 1 to 8, further comprising:

   - identifying a size and/or a size distribution of the luminescent particles present in dispersed form in the suspension.

10. The method according to claim 9, wherein the determination is carried out using a measuring method selected from: dynamic light scattering (DLS); electron microscopy, in particular transmission electron microscopy (TEM) and/or scanning electron microscopy (SEM); small angle X-ray diffraction (SAX); ultracentrifugation or laser diffraction measurement, or laser diffractometry.

11. The method according to any one of claims 1 to 10, furthermore comprising:

   - determining a dielectric constant of the material of the particles and/or determining a density of the material of the particles.

12. The method according to any one of claims 1 to 11, wherein the particle ensemble is a particle suspension filling the sample volume.

13. Use of at least one luminescent particle as a reference standard or as an assay reagent for a particle-based test, an absolute brightness being associated with the luminescent particle, which is determined according to the method according to any one of claims 1 to 12, and the particle-based test being selected from: enzyme immunoassay (EIA); enzyme-linked immunosorbent assay (ELISA); a cytometry analysis method; a flow cytometry analysis method; a particle-based test in the field of environmental analytics; a particle-based test in the field of bioanalytics; a particle-based test in the field of medical diagnostics; a particle-based test in the field of pharmaceutical active ingredient research; a particle-based test for therapy monitoring; a use of particles as a contrast medium; a use of quantum dot structures as fluorescence labels and/or as contrast media; a use of phosphorene and nanophosphorene, and/or the use of a particle emulsion.

14. A method for producing a standard set of luminescent particles, wherein luminescent particles are provided, the absolute brightness of the luminescent particles is determined according to the method according to any one of claims 1 to 12, and the particles are associated with the absolute brightness thus determined.

15. The method according to claim 14, wherein a set of different particles is provided, the particles differing from one another in the fluorophore and/or the concentration of the fluorophore and/or in the particle size.

## Revendications

1. Procédé de détermination d'une luminosité absolue d'une particule luminescente, comprenant :

    - fourniture d'un volume d'échantillonnage qui contient un ensemble de particules luminescentes ;
    - détermination d'un rendement quantique de photoluminescence absolu ou relatif de l'ensemble de particules dans le volume d'échantillonnage ;
    - détermination d'une section efficace d'absorption ou de l'efficacité d'absorption des particules dans le volume d'échantillonnage ;
    - détermination de la luminosité absolue d'une particule individuelle sur la base du rendement quantique de photoluminescence absolu déterminé, de la section efficace d'absorption ou de l'efficacité d'absorption, et d'un nombre de particules dans le volume d'échantillonnage, la luminosité absolue étant exprimée par un produit de la section efficace d'absorption d'une particule $\sigma_a$ [cm$^2$] et du rendement quantique $\Phi_f$ mesuré sur une dispersion de particules ;
    les particules luminescentes étant sélectionnées parmi des particules en suspension ou dispersées dans le volume d'échantillonnage dans la plage de grandeur de 5 nm à 5 000 $\mu$m, et le rendement quantique de photoluminescence absolu et/ou la section efficace d'absorption étant déterminé(e) au moyen d'une mesure à résolution de longueur d'onde d'une émission, d'une transmission et d'une réflexion.

2. Procédé selon la revendication 1, une sphère intégrante étant utilisée pour la détermination du rendement quantique de photoluminescence absolu et/ou de la section efficace d'absorption.

3. Procédé selon la revendication 1, la détermination du rendement quantique de photoluminescence absolu et/ou de la section efficace d'absorption s'effectuant au moyen de la photoacoustique.

4. Procédé selon l'une des revendications 1 à 3, la détermination du rendement quantique de photoluminescence relatif et/ou de la section efficace d'absorption s'effectuant relativement à un standard.

5. Procédé selon l'une des revendications 1 à 4, la détermination du rendement quantique de photoluminescence et/ou de la section efficace d'absorption s'effectuant pour des longueurs d'onde d'excitation dans le domaine UV, vis ou NIR, en particulier dans le domaine de 250 à 1 500 nm, en particulier dans le domaine de 300 nm à 850 nm.

6. Procédé selon l'une des revendications 1 à 5, la détermination du rendement quantique de photoluminescence et/ou de la section efficace d'absorption s'effectuant pour des longueurs d'onde d'émission dans le domaine vis ou NIR de 400 à 1 000 nm, typiquement dans le domaine de 300 nm à 1 600 nm, en particulier dans la plage de longueurs d'onde de 350 à 1 000 nm.

7. Procédé selon l'une des revendications 1 à 6, une section efficace d'absorption $\sigma_a$ d'une particule luminescente étant déterminée selon la formule

$$\sigma_a = \frac{\mu_a}{N},$$

$\sigma_a$ étant la section efficace d'absorption de la particule [cm$^2$] ;

$\mu_a$ étant le coefficient d'absorption [1/cm] de l'ensemble de particules dans le volume d'échantillonnage ; et

$N$ étant le nombre de particules par unité de volume,

le coefficient d'absorption $\mu_a$ de l'ensemble de particules étant déterminé sur la base de l'émission, de la transmission et de la réflexion de l'ensemble de particules dans le volume d'échantillonnage.

8. Procédé selon l'une des revendications 1 à 7, la luminosité absolue d'une particule individuelle étant définie comme le produit du rendement quantique de photoluminescence et de la section efficace d'absorption $\sigma_a$ [cm$^2$] d'une particule, et la luminosité absolue étant affectée à une particule luminescente ou à une pluralité des particules luminescentes.

9. Procédé selon l'une des revendications 1 à 8, comprenant en outre

- détermination d'une taille et/ou d'une distribution de taille des particules luminescentes dispersées dans la suspension.

10. Procédé selon la revendication 9, la détermination étant effectuée avec une méthode de mesure sélectionnée parmi : diffusion dynamique de la lumière (DLS) ; microscopie électronique, en particulier microscopie électronique en transmission (MET) et/ou microscopie électronique à balayage (MEB), diffusion des rayons X aux petits angles (SAX) ; mesure par ultracentrifugation ou diffraction laser ou respectivement diffractométrie laser.

11. Procédé selon l'une des revendications 1 à 10, comprenant en outre

- détermination d'une constante diélectrique de la matière des particules et/ou détermination d'une densité de la matière des particules.

12. Procédé selon l'une des revendications 1 à 11, l'ensemble de particules étant une suspension de particules qui remplit le volume d'échantillonnage.

13. Utilisation d'au moins une particule luminescente en tant que standard de référence ou en tant que réactif de détection pour un test à base de particules, une luminosité absolue étant affectée à la particule luminescente et étant déterminée selon le procédé selon l'une des revendications 1 à 12, et le test à base de particules étant sélectionné parmi : test dosage d'immunoabsorption par enzyme liée (EIA), test dosage immuno-enzymatiques (ELISA) ; un procédé d'analyse cytométrique ; un procédé d'analyse par cytométrie en flux ; un test à base de particules dans le domaine de l'analyse environnementale ; un test à base de particules dans le domaine de la bioanalyse ; un test à base de particules dans le domaine du diagnostic médical ; un test à base de particules dans le domaine de la recherche pharmaceutique sur les principes pharmaceutiques actifs ; un test à base de particules pour le contrôle thérapeutique ; une utilisation de particules comme milieu de contraste ; une utilisation de structures de points quantiques comme marqueur fluorescent et/ou comme milieu de contraste ; une utilisation de phosphores et de nanophosphores et/ou l'utilisation d'une émulsion de particules.

14. Procédé de fabrication d'un ensemble standard de particules luminescentes, des particules luminescentes étant fournies, la luminosité absolue des particules luminescentes étant déterminée selon le procédé selon l'une quelconque des revendications 1 à 12, et la luminosité absolue ainsi déterminée étant affectée aux particules.

15. Procédé selon la revendication 14, un ensemble de différentes particules étant fourni, les particules se distinguant en fluorophore et/ou en concentration de fluorophore et/ou en taille des particules.

Fig. 1A

Fig. 1B

Fig. 2

**Fig. 3**

**Fig. 4**

EP 2 634 560 B1

**Fig. 5**

EP 2 634 560 B1

**Fig. 6**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009006364 A1

- EP 2357465 A1

**In der Beschreibung aufgeführte Nicht-Patentliteratur**

- **WÜRTH C. ; GRABOLLE M. ; PAULI J. ; SPIELES M. ; RESCH-GENGER U.** *Analytical Chemistry,* 2011, vol. 83, 3431-3439

- **WÜRTH C. ; HOFFMANN K. ; BEHNKE T. ; OHNE-SORGE M. ; RESCH-GENGER U.** *Journal of Fluorescence,* 2011, vol. 21, 953-961
- Light scattering by small particles. Dover Publications, 1981